(19) Europäisches Patentamt
European Patent Office
Office européen des brevets

(11) Veröffentlichungsnummer: **0 073 931**
**B1**

(12) # EUROPÄISCHE PATENTSCHRIFT

(45) Veröffentlichungstag der Patentschrift: **13.02.85**

(51) Int. Cl.⁴: **C 07 D 249/04,** A 61 K 31/41

(21) Anmeldenummer: **82107026.5**

(22) Anmeldetag: **04.08.82**

(54) **Neue Anilino-1,2,3-triazol-Derivate, diese enthaltende Arzneimittel sowie Verfahren zu deren Herstellung und deren Verwendung.**

(30) Priorität: **03.09.81 DE 3134842**

(43) Veröffentlichungstag der Anmeldung: **16.03.83 Patentblatt 83/11**

(45) Bekanntmachung des Hinweises auf die Patenterteilung: **13.02.85 Patentblatt 85/7**

(84) Benannte Vertragsstaaten: **AT BE CH DE FR IT LI LU NL SE**

(56) Entgegenhaltungen:
**EP-A-0 054 416**
**DD-A-80 210**
**FR-A-2 388 557**
**GB-A-1 473 578**

(73) Patentinhaber: **BOEHRINGER INGELHEIM KG, D-6507 Ingelheim am Rhein (DE)**

(72) Erfinder: **Kummer, Werner, Dr., Georg-Scheuing-Strasse 15, D-6507 Ingelheim/Rhein (DE)**
Erfinder: **Köppe, Herbert, Dr., Neuweg 72, D-6507 Ingelheim/Rhein (DE)**
Erfinder: **Stähle, Helmut, Dr., Rotweinstrasse 23, D-6507 Ingelheim/Rhein (DE)**
Erfinder: **Reichl, Richard, Dr., Im Hippel 55, D-6535 Gau Algesheim (DE)**
Erfinder: **Kuhn, Franz Josef, Dr., BeethovenStrasse 11, D-6535 Gau-Algesheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 073 931 B1

BUNDESDRUCKEREI BERLIN

**Beschreibung**

Die Erfindung betrifft neue Anilino-1,2,3-triazol-Derivate der allgemeinen Formel

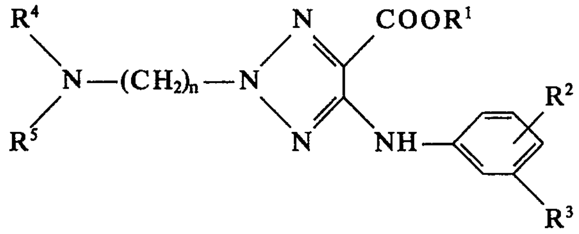

(I)

worin

$R^1$ eine gerade oder verzweigte Alkylgruppe mit 1 bis 4 Kohlenstoffatomen,
$R^2$ ein Wasserstoffatom, ein Halogenatom, eine Aminogruppe, eine Nitrogruppe, eine Alkoxigruppe mit 1 bis 3 Kohlenstoffatomen, eine Estergruppe $COOR^6$ oder eine Methylgruppe in 3- oder 4-Stellung,
$R^3$ ein Wasserstoffatom oder ein Halogenatom in 5-Stellung,
$R^4$ und $R^5$ Alkylgruppen mit 1 bis 4 Kohlenstoffatomen oder zusammen mit dem Stickstoffatom ein Piperidin-, Pyrrolidin- oder Morpholinring,
$R^6$ eine Alkylgruppe mit 1 bis 3 Kohlenstoffatomen und
n die Zahl 2 oder 3 bedeutet, sowie deren Säureadditionssalze mit organischen und anorganischen Säuren.

Bevorzugt sind Anilino-1,2,3-triazol-Derivate der allgemeinen Formel

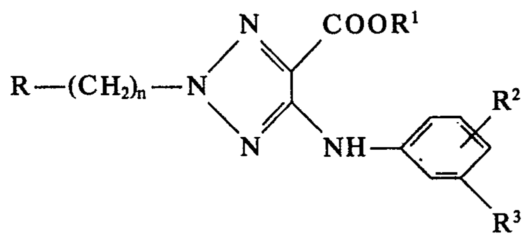

(Ia)

worin

$R^1$ eine gerade oder verzweigte Alkylgruppe mit 1 bis 4 Kohlenstoffatomen,
$R^2$ ein Wasserstoffatom, ein Chloratom in 3- oder 4-Stellung oder eine Methylgruppe, eine Methoxigruppe, eine Nitrogruppe, eine Aminogruppe, eine Methoxicarbonylgruppe, ein Fluor- oder Bromatom in 4-Stellung,
$R^3$ ein Wasserstoffatom oder ein Chloratom,
R eine Dimethylaminogruppe, eine Diethylaminogruppe oder ein Piperidyl-(1)- oder Morpholino-(4)-Ring und
n die Zahl 2 oder 3

bedeutet, sowie deren Säureadditionssalze.

Besonders bevorzugt sind Anilino-1,2,3-triazol-Derivate der allgemeinen Formel Ia, worin

$R^1$ eine Methylgruppe, eine Ethylgruppe oder eine n-Butylgruppe bedeutet und
R, $R^2$, $R^3$ und n die gleichen Bedeutungen haben, wie sie für die Verbindungen der allgemeinen Formel Ia gelten, sowie deren Säureadditionssalze.

Ganz besonders bevorzugt sind Anilino-1,2,3-triazol-Derivate der allgemeinen Formel Ia, worin

$R^1$ eine Methyl- oder n-Butylgruppe,
$R^2$ eine Methylgruppe oder ein Chlor- oder Bromatom in 4-Stellung,
$R^3$ ein Wasserstoffatom und
R eine Diethylamino- oder Dimethylaminogruppe und
n die Zahl 2 oder 3 bedeutet sowie deren Säureadditionssalze.

Die neuen Verbindungen können nach folgenden Verfahren hergestellt werden:

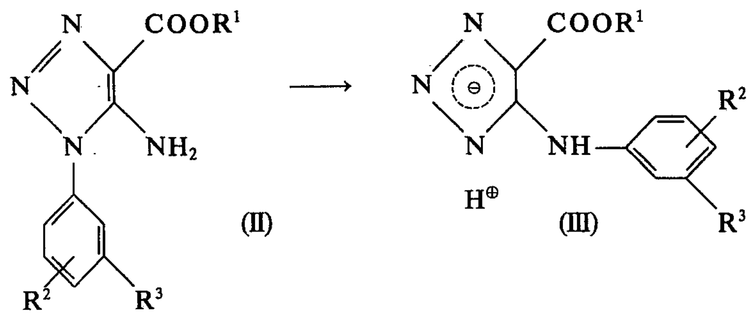

(II) (III)

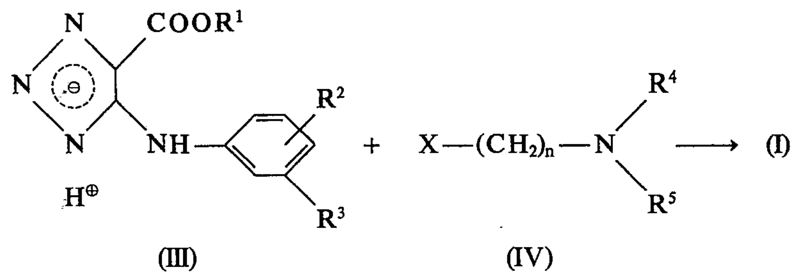

(III) (IV)

Man setzt ein Triazol-Derivat der Formel III mit einer Verbindung der Formel IV um, wobei $R^1$ bis $R^5$ und n die obigen Bedeutungen besitzen und X ein Halogenatom darstellt. Die Umsetzung wird bevorzugt in einem aprotischen polaren Lösungsmittel unter Zusatz einer Base, wie Natronlauge oder Soda durchgeführt. Die Reaktionszeiten und Reaktionstemperaturen sind von der Bedeutung der Reste $R^1$ bis $R^5$ und von X abhängig und sehr variabel.

Die Ausgangsverbindungen sind zum Teil bekannt und können leicht aus den entsprechenden 1-Phenyl-5-amino-1 H-1,2,3-triazol-4-carbonsäureester der allgemeinen Formel II durch Dimroth-Umlagerung erhalten werden. Diese Umlagerung findet oft bereits durch Erhitzen mit polaren Lösungsmitteln, insbesondere bei Basenzusatz statt, so daß die Reaktion von Verbindungen der allgemeinen Formel III und IV am vorteilhaftesten unter Einsatz von 1-Phenyl-5-amino-1 H-1,2,3-triazol-4-carbonsäureestern der allgemeinen Formel II ohne Isolierung der durch Umlagerung entstehenden Verbindungen der allgemeinen Formel III durchgeführt werden kann.

Die erfindungsgemäßen Anilino-1,2,3-triazole der allgemeinen Formel I sind Basen und können auf übliche Weise in ihre Säureadditionssalze überführt werden. Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Capronsäure, Valeriansäure, Oxalsäure, Malonsäure, Bernsteinsäure, Maleinsäure, Fumarsäure, Milchsäure, Weinsäure, Zitronensäure, Äpfelsäure, Benzoesäure, p-Hydroxybenzoesäure, p-Aminobenzoesäure, Phthalsäure, Zimtsäure, Salicylsäure, Ascorbinsäure, Methansulfonsäure, 8-Chlortheophyllin und dergleichen.

Die neuen Anilino-1,2,3-triazol-Derivate der allgemeinen Formel I besitzen sowohl als Basen als auch in Form ihrer Salze im Tierversuch wertvolle therapeutische Eigenschaften. Insbesondere wirken sie schlafpotenzierend, ohne den natürlichen Schlafrhythmus zu beeinflussen und fördern außerdem die Durchblutung. Die Toxizitäten sind im allgemeinen gering, wie durch Toxizitätsuntersuchungen an der Maus bei i.v.- und p.o.-Gabe festgestellt werden konnte.

Aufgrund dieser Wirkungen kommen die Verbindungen der allgemeinen Formel I als Wirkstoffe für Arzneimittel zur Behandlung von Durchblutungs- und Schlafstörungen in Frage. Die erfindungsgemäßen Wirkstoffe können enteral auch parenteral angewandt werden. Die Dosierung für die orale Anwendung liegt zwischen 0,05 und 50 mg/kg, bei i.v.-Gabe zwischen 0,01 und 10 mg vorzugsweise 1 bis 5 mg/kg.

Die neuen Anilino-1,2,3-triazol-Derivate der allgemeinen Formel I bzw. ihre Säureadditionssalze können auch mit andersartigen Wirkstoffen kombiniert werden. Geeignete galenische Darreichungsformen sind beispielsweise Tabletten, Kapseln, Zäpfchen, Lösungen oder Pulver; hierbei können zu deren Herstellung die üblicherweise verwendeten galenischen Hilfs-, Träger-, Spreng- oder Schmiermittel oder Substanzen zur Erzielung einer Depotwirkung Anwendung finden.

Die folgenden Beispiele erläutern die Erfindung, ohne sie zu beschränken.

## Herstellungsbeispiele

### Beispiel 1

2-(3-Piperidyl-(1)-propyl)-5-(4-tolylamino)-2 H-1,2,3-triazol-4-carbonsäure-n-butylester-hydrochlorid

8,2 g (0,03 Mol) 1-(4-Tolyl)-5-amino-1 H-1,2,3-triazol-4-carbonsäure-n-butylester, 100 ml absolutes Dimethylformamid und 14,6 g (0,09 Mol) 3-Chlorpropyl-piperidin-(1) werden 4 Stunden bei 100°C gerührt. Anschließend wird die Lösung im Vakuum eingedampft, der Rückstand in 2 N Salzsäure gelöst und bei aufsteigendem pH-Wert fraktioniert mit Ether ausgeschüttelt. Die Etherphasen von pH 5 werden isoliert eingeengt, der Rückstand in Ethanol gelöst und mit etherischer Salzsäure das Hydrochlorid ausgefällt. Dieses wird aus Alkohol/Ether fraktioniert umgefällt.

Man erhält 1,9 g der gewünschten Verbindung, entsprechend 14,6% der Theorie, mit einem Schmelzpunkt von 148°C.

### Beispiel 2

2-(3-Dimethylamino-propyl)-5-(4-bromphenylamino)-2 H-1,2,3-triazol-4-carbonsäure-n-butylester-hydrochlorid

8,5 g (0,025 Mol) 1-(4-Bromphenyl)-5-amino-1 H-1,2,3-triazol-4-carbonsäure-n-butylester, 100 ml absolutes Dimethylformamid, und 9,1 g (0,075 Mol) 3-(N,N-Dimethylamino)-propylchlorid-(1) werden wie in Beispiel 1 umgesetzt und aufgearbeitet. Man erhält 1,5 g der gewünschten Verbindung, entsprechend 13% der Theorie, mit einem Schmelzpunkt von 158°C.

### Beispiel 3

2-(2-Dimethylamino-ethyl)-5-(4-chlorphenylamino)-2 H-1,2,3-triazol-4-carbonsäure-n-butylester-hydrochlorid

6,0 g (0,02 Mol) 1-(4-Chlorphenyl)-5-amino-1 H-1,2,3-triazol-4-carbonsäure-n-butylester, 100 ml absolutes Dimethylformamid und 4,3 g (0,04 Mol) 2-(Dimethylamino)-ethyl-chlorid-(1) werden wie in Beispiel 1 umgesetzt, das Reaktionsgemisch eingedampft, in 2 N Salzsäure und Wasser gelöst und bei steigendem pH-Wert mit Ether ausgeschüttelt. Nach dem Eindampfen der im Dünnschichtchromatogramm reinen Etherphasen wird der Rückstand in wenig Isopropanol gelöst, mit etherischer Salzsäure angesäuert und mit Essigsäureethylester ausgefällt. Nach Umkristallisieren aus Ethanol erhält man 0,8 g, entsprechend 9,9% der Theorie, der gewünschten Verbindung mit einem Schmelzpunkt von 171°C.

### Beispiel 4

2-(2-Diethylamino-ethyl)-5-(4-chlorphenylamino)-2 H-1,2,3-triazol-4-carbonsäure-n-butylester-hydrochlorid

Zu einer Lösung von 2 g (0,05 Mol) Natriumhydroxid in 40 ml destilliertem Wasser gibt man 8,4 g (0,025 Mol) 1-Acetyl-5-(4-chlorphenylamino)-1 H-1,2,3-triazol-4-carbonsäure-n-butylester und rührt bei Raumtemperatur 30 Minuten. Zu der hierbei entstehenden de-acetylierten Verbindung gibt man 60 ml Toluol, 10 mg 18-Crown-6-ether und tropft innerhalb von 2 Minuten bei Raumtemperatur 3,3 g (0,024 Mol) Diethylamino-ethylchlorid zu. Dann wird bei Raumtemperatur 1 Stunde gerührt, nochmals 3,3 g (0,024 Mol) Diethylamino-ethylchlorid zugetropft, 1 Stunde bei 35—40°C und 1 Stunde bei 60°C gerührt.

Anschließend werden Toluol- und Wasserphase voneinander getrennt, die Toluolphase nacheinander mit Wasser und Wasser/2 N Salzsäure ausgeschüttelt. Dabei bilden sich 3 Phasen: Toluol, Wasser und Öl. Das Öl wurde abgetrennt, in 2 N Salzsäure gelöst und mehrmals mit Ether ausgeschüttelt. Toluol- und Etherphasen werden verworfen. Die Wasserphasen gibt man zusammen und schüttelt bei aufsteigendem pH-Wert mit Ether aus. Die Etherphase von pH 5 wird über Natriumsulfat getrocknet, im Vakuum eingedampft und der Rückstand in wenig Essigsäure-ethylester gelöst.

Mit etherischer Salzsäure wird das Hydrochlorid ausgefällt und aus Isopropanol umkristallisiert.

Man erhält 1,6 g, entsprechend 14,5% der Theorie von der gewünschten Verbindung mit einem Schmelzpunkt von 170°C.

Beispiel 5

2-(2-Diethylamino-ethyl)-5-(4-chlorphenylamino)-2 H-1,2,3-triazol 4-carbonsäure ethylester hydrochlorid

5,4 g (0,02 Mol) 1-(4-Chlorphenyl)-5-amino-1 H-1,2,3-triazol-4-carbonsäure-ethylester, 100 ml absolutes Dimethylformamid und 5,4 g (0,04 Mol) Diethylamino-ethylchlorid werden wie in Beispiel 3 umgesetzt und aufgearbeitet.

Man erhält 1,4 g, entsprechend 17,4% der Theorie, der gewünschten Verbindung mit einem Schmelzpunkt von 178°C.

Beispiel 6

2-(3-Morpholino-(4)-propyl)-5-(4-chlorphenylamino)-2 H-1,2,3-triazol-4-carbonsäure-ethylester-hydrochlorid

5,4 g (0,02 Mol) 1-(4-Chlorphenyl)-5-amino-1 H-1,2,3-triazol-4-carbonsäure-ethylester, 100 ml absolutes Dimethylformamid und 8,9 g (0,05 Mol) 4-(1-Chlorpropyl)-morpholin-(1) werden wie in Beispiel 1 umgesetzt, die Lösung eingedampft und der Rückstand in 2 N Salzsäure und Wasser gelöst. Man schüttelt bei steigendem pH-Wert bis pH 9 mit Ether aus, gibt die im Dünnschichtchromatogramm reinen Etherphasen zusammen, trocknet über Natriumsulfat und dampft die Lösung im Vakuum ein. Der Rückstand wird in Chloroform gelöst, mit etherischer Salzsäure das Hydrochlorid hergestellt und mit Ether ausgefällt. Man kristallisiert zweimal aus Alkohol um und erhält 0,8 g, entsprechend 9,3% der Theorie der gewünschten Verbindung mit einem Schmelzpunkt von 223°C.

Beispiel 7

2-(2-Diethylamino-ethyl)-5-(4-chlorphenylamino)-2 H-1,2,3-triazol-4-carbonsäure-methylester-hydrochlorid

5,1 g (0,02 Mol) 1-(4-Chlorphenyl)-5-amino-1 H-1,2,3-triazol-4-carbonsäure-methylester, 50 ml absolutes Dimethylformamid und 2,1 g (0,02 Mol) wasserfreie Soda werden bei Raumtemperatur 5 Minuten gerührt. Man fügt 2,7 g (0,02 Mol) Diethylamino-ethylchlorid hinzu, rührt 3 Stunden bei 100°C und dampft das Lösungsmittel im Vakuum ab. Der Rückstand wird in 2 N Salzsäure gelöst, bei pH 2 bis pH 6 fraktioniert mit Ether ausgeschüttelt, die im Dünnschichtchromatogramm reinen Etherphasen vereint über Natriumsulfat getrocknet und im Vakuum eingeengt. Man löst den Rückstand in wenig Methanol, bildet durch Zugabe von etherischer Salzsäure das Hydrochlorid und fällt es mit Ether aus.

Man erhält 2,4 g, entsprechend 30,9% der Theorie der gewünschten Verbindung mit einem Schmelzpunkt von 18°C.

Beispiel 8

2-(3-Diethylamino-propyl)-5-(4-chlorphenylamino)-2 H-1,2,3-triazol-4-carbonsäure-methylester-hydrochlorid

5,1 g (0,02 Mol) 1-(4-Chlorphenyl)-5-amino-1 H-1,2,3-triazol-4-carbonsäure-methylester, 100 ml absolutes Dimethylformamid und 7,5 g (0,05 Mol) 3-Diethylamino-propylchlorid-(1) werden analog zu Beispiel 1 umgesetzt, das Reaktionsgemisch im Vakuum eingedampft, der Rückstand in 2 N Salzsäure gelöst und bei steigendem pH-Wert mit Ether fraktioniert ausgeschüttelt. Nach dem Eindampfen der im Dünnschichtchromatogramm identischen reinen Etherphasen wird der Rückstand in Essigsäure-ethylester gelöst und mit etherischer Salzsäure das Hydrochlorid ausgefällt. Nach zweimaligem Umkristallisieren erhält man 1,1 g, entsprechend 13,7% der Theorie an gewünschter Verbindung, mit einem Schmelzpunkt von 181°C.

Beispiel 9

2-(3-Dimethylamino-propyl)-5-(4-chlorphenylamino)-2 H-1,2,3-triazol-4-carbonsäure-methylester-hydrochlorid

5,1 g (0,02 Mol) 1-(4-Chlorphenyl)-5-amino-1 H-1,2,3-triazol-carbonsäure-methylester, 100 ml absolutes Dimethylformamid und 6,1 g (0,05 Mol) 3-Dimethylamino-propylchlorid-(1) werden wie in Beispiel 8 umgesetzt und aufgearbeitet.

Man erhält 1,4 g (18,7% der Theorie) der gewünschten Verbindung mit einem Schmelzpunkt von 197°C.

## Beispiel 10

### 2-(3-Morpholino-(4)-propyl)-5-(4-chlorphenylamino)-2 H-1,2,3-triazol-4-carbonsäure-methylester-hydrochlorid

7,6 g (0,03 Mol) 1-(4-Chlorphenyl)-5-amino-1 H-1,2,3-triazol-4-carbonsäure-methylester, 100 ml absolutes Dimethylformamid und 16,2 g (0,09 Mol) 3-Morpholino-(4)-propylchlorid-(1) werden wie in Beispiel 1 umgesetzt und aufgearbeitet.

Man erhält 1,0 g (8 % der Theorie) an gewünschter Verbindung mit einem Schmelzpunkt von 217°C.

## Beispiel 11

### 2-(2-Diethylamino-ethyl)-5-(4-fluorphenylamino)-2 H-1,2,3-triazol-4-carbonsäure-methylester-hydrochlorid

4,7 g (0,02 Mol) 1-(4-Fluorphenyl)-5-amino-1 H-1,2,3-triazol-4-carbonsäure-methylester, 70 ml absolutes Dimethylformamid und 8,1 g (0,06 Mol) Diethylamino-ethylchlorid werden wie in Beispiel 1 umgesetzt, das Reaktionsgemisch im Vakuum eingeengt und der Rückstand in 2 N Salzsäure gelöst. Man schüttelt mit Chloroform aus, filtriert über Aktivkohle, trocknet über Natriumsulfat und destilliert das Chloroform im Vakuum ab. Nach Umkristallisation aus Isopropanol erhält man 1,5 g (20,2 % der Theorie) mit einem Schmelzpunkt von 179°C.

## Beispiel 12

### 2-(2-Diethylamino-ethyl)-5-(4-bromphenylamino)-2 H-1,2,3-triazol-4-carbonsäure methylester-hydrochlorid

6,5 g (0,022 Mol) 1-(4-Bromphenyl)-5-amino-1 H-1,2,3-triazol-4-carbonsäure-methylester, 100 ml Dimethylformamid und 8,9 g (0,066 Mol) 2-Diethylamino-ethylchlorid-(1) werden wie in Beispiel 11 umgesetzt und aufgearbeitet.

Man erhält eine Ausbeute von 2,3 g (24,2 % der Theorie) der gewünschten Verbindung mit einem Schmelzpunkt von 199°C.

## Beispiel 13

### 2-(2-Diethylamino-ethyl)-5-(3-chlor-4-methyl-phenylamino)-2 H-1,2,3-triazol-4-carbonsäure-methylester-hydrochlorid

5 g (0,019 Mol) 1-(3-Chlor-4-methyl-phenyl)-5-amino-1 H-1,2,3-triazol-4-carbonsäure-methylester, 100 ml absolutes Dimethylformamid und 7,7 g (0,057 Mol) 2-Diethylaminoethylchlorid-(1) werden wie in Beispiel 9 umgesetzt und aufgearbeitet.

Man erhält eine Ausbeute von 1,0 g, entsprechend 13,1 % der Theorie der gewünschten Verbindung mit einem Schmelzpunkt von 182°C.

## Beispiel 14

### 2-(2-Diethylamino-ethyl)-5-(3,4-dichlorphenylamino)-2 H-1,2,3-triazol-4-carbonsäure-methylester-maleat

8,6 g (0,03 Mol) 1-(3,4-Dichlorphenyl)-5-amino-1 H-1,2,3-triazol-4-carbonsäure-methylester, 100 ml absolutes Dimethylformamid, 8,1 g (0,06 Mol) 2-Diethylamino-ethylchlorid-(1) und 10 mg 18-Crown-6-ether werden wie in Beispiel 1 umgesetzt, das Reaktionsgemisch im Vakuum eingedampft und der Rückstand mit 2 N Natronlauge verrührt. Man schüttelt mit Chloroform aus, saugt langsam über Aluminiumoxid und Kohle ab und engt im Vakuum ein. Der Rückstand wird mit Ethanol gelöst, mit alkoholischer Maleinsäure das Salz gebildet und mit Ether ausgefällt.

Man erhält eine Ausbeute von 1,0 g, entsprechend 6,6 % der Theorie, der gewünschten Verbindung mit einem Schmelzpunkt von 135°C.

Beispiel 15

2-(3-Diethylamino-propyl)-5-(3,4-dichlorphenylamino)-2 H-1,2,3-triazol-4-carbonsäure-methylester-oxalat

5,8 g (0,02 Mol) 1-(3,4-Dichlorphenyl)-5-amino-1 H-1,2,3-triazol-4-carbonsäure-methylester, werden in 80 ml absolutem Dimethylformamid vorgelegt und 0,8 g 55 %ige Natrium-Hydrid-Dispersion portionsweise zugefügt. Dabei bildet sich eine klare Lösung und die Temperatur steigt von 20° auf 25°C an. Nun fügt man 3 g (0,02 Mol) 3-Diethylamino-propylchlorid-(1) zu und rührt 16 Stunden bei Raumtemperatur. Das Lösungsmittel wird nun im Vakuum abdestilliert, der Rückstand mit 2 N Salzsäure verrührt und die ungelösten Bestandteile abgesaugt. Das Filtrat wird bei steigendem pH-Wert mit Ether fraktioniert ausgeschüttelt, die im Dünnschichtchromatogramm reinen Etherphasen zusammengegeben, über Natriumsulfat getrocknet und im Vakuum eingeengt. Der Rückstand wird in Ethanol gelöst, mit alkoholischer Oxalsäure das Salz gefällt und aus Ethanol umkristallisiert.

Man erhält eine Ausbeute von 1,2 g (12,3 % der Theorie) der gewünschten Verbindung mit einem Schmelzpunkt von 148°C.

Beispiel 16

2-(2-Diethylamino-ethyl)-5-(3,5-dichlorphenylamino)-2 H-1,2,3-triazol-4-carbonsäure-methylester-hydrochlorid

5,8 g (0,02 Mol) 1-(3,5-Dichlorphenyl)-5-amino-1 H-1,2,3-triazol-4-carbonsäure-methylester, 80 ml absolutes Dimethylformamid, 0,8 g 55 %ige Natrium-Hydrid-Dispersion, 2,7 g (0,02 Mol) Diethylamino-ethylchlorid-(1) und 10 mg 18-Crown-6-ether werden wie in Beispiel 15 umgesetzt und aufgearbeitet.

Man erhält eine Ausbeute von 1,5 g (15,8 % der Theorie) der gewünschten Verbindung mit einem Schmelzpunkt von 188°C.

Beispiel 17

2-(3-Diethylamino-propyl)-5-(3,5-dichlorphenylamino)-2 H-1,2,3-triazol-4-carbonsäure-methylester-hydrochlorid

5,8 g (0,02 Mol) 1-(3,5-Dichlorphenyl)-5-amino-1 H-1,2,3-triazol-4-carbonsäure-methylester, 80 ml absolutes Dimethylformamid, 0,8 g Natrium-Hydrid-Dispersion und 3,0 g (0,02 Mol) 3-Diethylamino-propylchlorid-(1) werden wie in Beispiel 15 umgesetzt und aufgearbeitet.

Man erhält eine Ausbeute von 0,9 g (9,2 % der Theorie) der gewünschten Verbindung mit einem Schmelzpunkt von 158°C.

Beispiel 18

2-(2-Dimethylamino-ethyl)-5-(3,4-dichlorphenylamino)-2 H-1,2,3-triazol-4-carbonsäure-methylester-hydrochlorid

5,8 g (0,02 Mol) 1-(3,4-Dichlorphenyl)-5-amino-1 H-1,2,3-triazol-4-carbonsäure-methylester werden mit 2,3 g (0,02 Mol) Kalium-tert.-butylat in 100 ml absolutem Dimethylformamid 1 Stunde bei 100°C gerührt, dann auf 20°C abgekühlt und mit 2,2 g (0,02 Mol) 2-Dimethylamino-ethylchlorid-(1) versetzt. Man rührt 3 Stunden bei 100° bis 120°C, destilliert das Lösungsmittel im Vakuum ab und löst in 2 N Salzsäure. Sodann wird mit Ether fraktioniert ausgeschüttelt, die gewünschten Etherphasen getrocknet, im Vakuum eingeengt und der Rückstand mit Ethanol gelöst. Mit etherischer HCl wird das Salz gebildet, mit Ether ausgefällt und die Kristalle zweimal aus Alkohol umkristallisiert.

Man erhält eine Ausbeute von 0,3 g (4 % der Theorie) der gewünschten Verbindung mit einem Schmelzpunkt von 200°C.

Beispiel 19

2-(2-Dimethylamino-ethyl)-5-(3,5-dichlorphenylamino)-2 H-1,2,3-triazol-4-carbonsäure-methylester-hydrochlorid

5,8 g (0,02 Mol) 1-(3,5-Dichlorphenyl)-5-amino-1 H-1,2,3-triazol-4-carbonsäure-methylester werden in 100 ml absolutem Dimethylformamid mit 2,3 g (0,02 Mol) Kalium-tert.-butylat und 2,2 g (0,02 Mol) 2-Dimethylamino-ethylchlorid-(1) wie in Beispiel 18 umgesetzt und aufgearbeitet.

Man erhält 0,3 g (4 % der Theorie) der gewünschten Verbindung mit einem Schmelzpunkt von 212 °C.

## Beispiel 20

### 2-(2-Dimethylamino-ethyl)-5-(4-chlorphenylamino)-2 H-1,2,3-triazol-4-carbonsäure-methylester-hydrochlorid

5,1 g (0,02 Mol) 1-(4-Chlorphenyl)-5-amino-1 H-1,2,3-triazol-4-carbonsäure-methylester werden in 100 ml absolutem Dimethylformamid mit 2,3 g (0,02 Mol) Kalium-tert.-butylat und 2,2 g (0,02 Mol) 2-Dimethylamino-ethylchlorid-(1) wie in Beispiel 18 umgesetzt und aufgearbeitet.

Man erhält eine Ausbeute von 0,8 g (11 % der Theorie) der gewünschten Verbindung mit einem Schmelzpunkt von 205°C.

## Beispiel 21

### 2-(2-Diethylamino-ethyl)-5-(4-methoxiphenylamino)-2 H-1,2,3-triazol-4-carbonsäure-methylester-hydrochlorid

10,0 g (0,04 Mol) 1-(4-Methoxiphenyl)-5-amino-1 H-1,2,3-triazol-4-carbonsäure-methylester werden in 200 ml absolutem Dimethylformamid mit 4,5 g (0,04 Mol) Kalium-tert.-butylat und 5,4 g (0,04 Mol) 2-Diethylamino-ethylchlorid-(1) wie in Beispiel 18 umgesetzt und aufgearbeitet.

Man erhält eine Ausbeute von 1,2 g (7,8 % der Theorie) der gewünschten Verbindung mit einem Schmelzpunkt von 145°C.

## Beispiel 22

### 2-(2-Diethylamino-ethyl)-5-phenylamino-2 H-1,2,3-triazol-4-carbonsäure-methylester-hydrochlorid

8,7 g (0,04 Mol) 1-Phenyl-5-amino-1 H-1,2,3-triazol-4-carbonsäure-methylester werden in 150 ml absolutem Dimethylformamid mit 4,5 g (0,04 Mol) Kalium-tert.-butylat und 5,5 g (0,04 Mol) 2-Diethylamino-ethylchlorid-(1) wie in Beispiel 18 umgesetzt und aufgearbeitet, jedoch wird die Base in Isopropanol gelöst, mit etherischer HCl das Salz gebildet und mit Essigester ausgefällt. Die Kristalle werden aus Alkohol/Ether umgefällt.

Man erhält eine Ausbeute von 2,3 g (16 % der Theorie) der gewünschten Verbindung mit einem Schmelzpunkt von 150°C.

## Beispiel 23

### 2-(2-Dimethylamino-ethyl)-5-(3-chlor-4-methyl-phenylamino)-2 H-1,2,3-triazol-4-carbonsäure-methylester-hydrochlorid

5,4 g (0,02 Mol) 1-(3-Chlor-4-methyl-phenyl)-5-amino-1 H-1,2,3-triazol-4-carbonsäure-methylester werden in 100 ml absolutem Dimethylformamid mit 2,3 g (0,02 Mol) Kalium-tert.-butylat und 2,2 g (0,02 Mol) 2-Dimethylamino-ethylchlorid-(1) wie in Beispiel 22 bzw. 18 umgesetzt und aufgearbeitet.

Man erhält eine Ausbeute von 0,7 g (9,35 % der Theorie) der gewünschten Verbindung mit einem Schmelzpunkt von 191°C.

## Beispiel 24

### 2-(2-Diethylamino-ethyl)-5-(4-nitrophenylamino)-2 H-1,2,3-triazol-4-carbonsäure-methylester-hydrochlorid

43,4 g (0,165 Mol) 1-(4-Nitrophenyl)-5-amino-1 H-1,2,3-triazol-4-carbonsäure-methylester werden in 300 ml absolutem Dimethylformamid mit 18,5 g (0,165 Mol) Kalium-tert.-butylat und 22,35 g (0,165 Mol) 2-Diethylaminoethylchlorid-(1) wie in Beispiel 18 umgesetzt, das Lösungsmittel abdestilliert und der Rückstand in 2 N Salzsäure gelöst. Man fällt mit 2 N Natronlauge fraktioniert aus. Die Base wird abgesaugt, in Alkohol gelöst und mit etherischer HCl das Salz gefällt.

Man erhält eine Ausbeute von 4,6 g (7 % der Theorie) der gewünschten Verbindung mit einem Schmelzpunkt von 195°C.

## Beispiel 25

**2-(2-Diethylamino-ethyl)-5-(4-aminophenylamino)-2 H-1,2,3-triazol-4-carbonsäure-methylester-dihydrochlorid**

4,2 g (0,01 Mol) 2-(2-Diethylamino-ethyl)-5-(4-nitrophenylamino)-2 H-1,2,3-triazol-4-carbonsäure-methylester werden in 80 ml Methanol bei 20°C und einem Druck von 5 bar Wasserstoff im Autoklaven unter Zusatz von Raney-Nickel hydriert. Der Katalysator wird nach beendeter Wasserstoffaufnahme abgesaugt, das Filtrat im Vakuum eingeengt und der Rückstand in Alkohol gelöst. Mit etherischer HCl wird das Salz gefällt und aus Alkohol/Ether umgefällt.

Man erhält eine Ausbeute von 0,4 g (10% der Theorie) der gewünschten Verbindung mit einem Schmelzpunkt von 162°C.

## Beispiel 26

**2-(2-Diethylamino-ethyl)-5-(4-methoxicarbonylphenylamino)-2 H-1,2,3-triazol-4-carbonsäure-methylester-hydrochlorid**

11,0 g (0,04 Mol) 1-(4-Methoxicarbonylphenyl)-5-amino-1 H-1,2,3-triazol-4-carbonsäure-methylester werden in 150 ml absolutem Dimethylformamid mit 4,5 g (0,04 Mol) Kalium-tert.-butylat und 5,4 g (0,04 Mol) 2-Diethylamino-ethylchlorid-(1) wie in Beispiel 22 umgesetzt und aufgearbeitet.

Man erhält eine Ausbeute von 2,4 g (14,6% der Theorie) der gewünschten Verbindung mit einem Schmelzpunkt von 194°C.

Nach dem in den Beispielen 1–26 beschriebenen Verfahren wurden ferner die folgenden Endprodukte der allgemeinen Formel I erhalten:

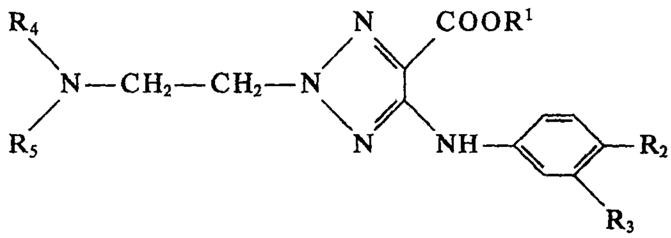

| Beispiel Nr. | $R_4$ N $R_5$ | $R_1$ | $R_2$ | $R_3$ | Fp. °C (Hydrochlorid) |
|---|---|---|---|---|---|
| 27 | $(C_2H_5)_2N-$ | $-CH(CH_3)_2$ | Cl | H | 152 |
| 28 | $(C_2H_5)_2N-$ | $-CH(CH_3)_2$ | Cl | Cl | 207 |
| 29 | 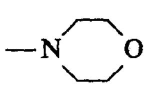 | $-CH(CH_3)_2$ | Cl | Cl | 237 |
| 30 | $(C_2H_5)_2N-$ | $-\overset{\overset{CH_3}{\vert}}{C}H-CH_2-CH_3$ | H | Cl | 168 |
| 31 | $(C_2H_5)_2N-$ | $-\overset{\overset{CH_3}{\vert}}{C}H-CH_2-CH_3$ | Cl | Cl | 210 |
| 32 | 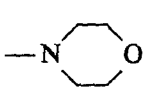 | $-\overset{\overset{CH_3}{\vert}}{C}H-CH_2-CH_3$ | Cl | Cl | 182 |
| 33 | $(C_2H_5)_2N-$ | $-CH(CH_3)_2$ | H | Br | |
| 34 | 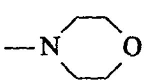 | $-CH(CH_3)_2$ | H | Br | |
| 35 | $(C_2H_5)_2N-$ | $-\overset{\overset{CH_3}{\vert}}{C}H-CH_2-CH_3$ | H | Br | |

Fortsetzung

| Beispiel Nr. | $R_4$ N $R_5$ | $R_1$ | $R_2$ | $R_3$ | Fp. °C (Hydrochlorid) |
|---|---|---|---|---|---|
| 36 | 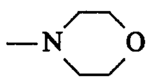 | 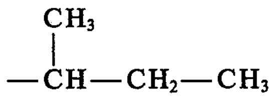 | H | Br | |
| 37 | $(C_2H_5)_2N—$ | $—CH_2—CH(CH_3)_2$ | H | Br | |
| 38 | $(C_2H_5)_2N—$ | $—CH_2—CH(CH_3)_2$ | Cl | Cl | |
| 39 | 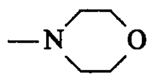 | $—CH_2—CH(CH_3)_2$ | H | Br | |
| 40 | 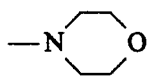 | $—CH_2—CH(CH_3)_2$ | Cl | Cl | |
| 41 | $(C_2H_5)_2N—$ | t-butyl | Cl | Cl | 200 |

## Formulierungsbeispiele

### Beispiel A

Dragées

| | | |
|---|---|---|
| Wirkstoff gemäß Erfindung | | 5 mg |
| Milchzucker | | 65 mg |
| Maisstärke | | 130 mg |
| sek. Calciumphosphat | | 40 mg |
| lösliche Stärke | | 3 mg |
| Magnesiumstearat | | 3 mg |
| kolloidale Kieselsäure | | 4 mg |
| | insgeamt | 250 mg |

Herstellung

Der Wirkstoff wird mit einem Teil der Hilfsstoffe vermischt, intensiv mit einer wäßrigen Lösung der löslichen Stärke durchgeknetet und in üblicher Weise mit Hilfe eines Siebes granuliert. Das Granulat wird mit dem Rest der Hilfsstoffe vermischt und zu Dragéekernen von 250 mg Gewicht verpreßt, die dann in üblicher Weise mit Hilfe von Zucker, Talkum und Gummi arabicum dragiert werden.

### Beispiel B

Ampullen

| | | |
|---|---|---|
| Wirkstoff gemäß Erfindung | | 1,0 mg |
| Natriumchlorid | | 18,0 mg |
| dest. Wasser | ad | 2,0 ml |

Herstellung

Wirkstoff und Natriumchlorid werden in Wasser gelöst und unter Stickstoff in Glasampullen abgefüllt.

Beispiel C

Tropfen

| | | |
|---|---|---|
| Wirkstoff gemäß Erfindung | | 0,02 g |
| p-Hydroxybenzoesäuremethylester | | 0,07 g |
| p-Hydroxybenzoesäurepropylester | | 0,03 g |
| entmineralisiertes Wasser | ad | 100 ml |

**Patentansprüche**

1. Neue Anilino-1,2,3-triazol-Derivate der allgemeinen Formel

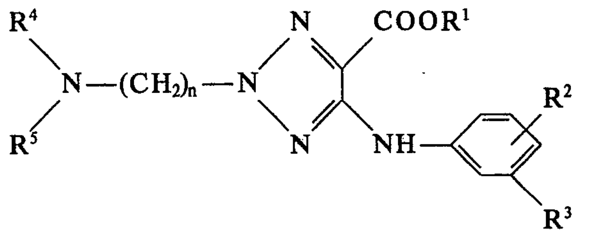

(I)

worin

$R^1$ eine gerade oder verzweigte Alkylgruppe mit 1 bis 4 Kohlenstoffatomen,
$R^2$ ein Wasserstoffatom, ein Halogenatom, eine Aminogruppe, eine Nitrogruppe, eine Alkoxigruppe mit 1 bis 3 Kohlenstoffatomen, eine Estergruppe $COOR^6$ oder eine Methylgruppe in 3- oder 4-Stellung,
$R^3$ ein Wasserstoffatom oder ein Halogenatom in 5-Stellung,
$R^4$ und $R^5$ Alkylgruppen mit 1 bis 4 Kohlenstoffatomen oder zusammen mit dem Stickstoffatom ein Piperidin-, Pyrrolidin- oder Morpholinring,
$R^6$ eine Alkylgruppe mit 1 bis 3 Kohlenstoffatomen und
n die Zahl 2 oder 3

bedeutet, sowie deren Säureadditionssalze.

2. Anilino-1,2,3-triazol-Derivate nach Anspruch 1 der allgemeinen Formel

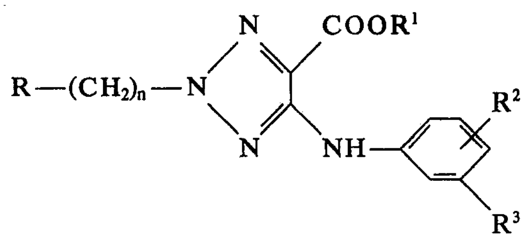

(Ia)

worin

$R^1$ eine gerade oder verzweigte Alkylgruppe mit 1 bis 4 Kohlenstoffatomen,
$R^2$ ein Wasserstoffatom, ein Chloratom in 3- oder 4-Stellung oder eine Methylgruppe, eine Methoxigruppe, eine Nitrogruppe, eine Aminogruppe, eine Methoxicarbonylgruppe, ein Fluor- oder Bromatom in 4-Stellung,
$R^3$ ein Wasserstoffatom oder ein Chloratom,
R eine Dimethylaminogruppe, eine Diethylaminogruppe oder ein Piperidyl-(1)- oder Morpholino-(4)-Ring und
n die Zahl 2 oder 3

bedeutet, sowie deren Säureadditionssalze.

3. Anilino-1,2,3-triazol-Derivate nach Anspruch 2 der allgemeinen Formel Ia, worin

$R^1$ eine Methylgruppe, eine Ethylgruppe oder eine n-Butylgruppe bedeutet und
R, $R^2$, $R^3$ und n die in Anspruch 2 angegebenen Bedeutungen besitzen sowie deren Säureadditionssalze.

4. Anilino-1,2,3-triazol-Derivate nach Anspruch 3 der allgemeinen Formel Ia, worin

$R^1$ eine Methyl- oder n-Butylgruppe,
$R^2$ eine Methylgruppe oder ein Chlor- oder Bromatom in 4-Stellung,
$R^3$ ein Wasserstoffatom und
R eine Diethylamino- oder Dimethylaminogruppe und
n die Zahl 2 oder 3

bedeutet sowie deren Säureadditionssalze.

5. Verfahren zur Herstellung von Verbindungen nach den Ansprüchen 1 bis 4, dadurch gekennzeichnet, daß man ein Triazol-Derivat der allgemeinen Formel

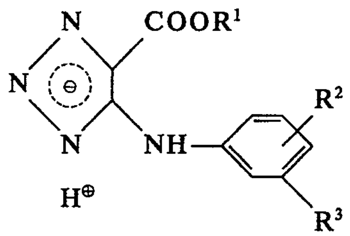

(III)

worin $R^1$ bis $R^3$ die oben genannten Bedeutungen besitzen, mit einer Verbindung der allgemeinen Formel

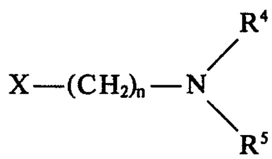

(IV)

worin $R^4$, $R^5$ und n wie oben angegeben definiert sind und X ein Halogenatom bedeutet, umsetzt und die Verbindung gegebenenfalls in ein Säureadditionssalz überführt.

6. Verfahren nach Anspruch 5, dadurch gekennzeichnet, daß man die Umsetzung in einem aprotischen, polaren Lösungsmittel und in Gegenwart einer Base durchführt.

7. Pharmazeutische Zubereitungen, dadurch gekennzeichnet, daß sie eine oder mehrere Verbindungen nach einem der Ansprüche 1 bis 4 neben üblichen Hilfs- und Trägerstoffen enthalten.

8. Verfahren zur Herstellung pharmazeutischer Zubereitungen nach Anspruch 7, dadurch gekennzeichnet, daß man eine oder mehrere Verbindungen nach einem der Ansprüche 1 bis 4 mit üblichen Hilfs-, Träger-, Spreng- oder Schmiermitteln bzw. Substanzen zur Erzielung einer Depotwirkung zu Tabletten, Kapseln, Zäpfchen, Pulver oder Lösungen formuliert.

9. Verbindungen nach einem der Ansprüche 1 bis 4 zur Verwendung bei der Behandlung von Durchblutungs- und Schlafstörungen.

10. Verwendung von Verbindungen nach einem der Ansprüche 1 bis 4 zur Herstellung von Arzneimitteln.

**Claims**

1. New anilino-1,2,3-triazol derivatives of the general formula

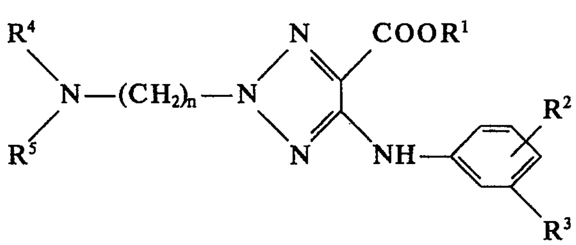

(I)

in which

$R^1$ is a straight chain or branched chain alkyl group having 1 to 4 carbon atoms,
$R^2$ is a hydrogen atom, a halogen atom, an amino group, a nitro group, an alkoxy group with 1 to 3 carbon atoms, an ester group $COOR^6$ or a methyl group attached to the 3 or 4 position,
$R^3$ is a hydrogen atom or a halogen atom attached to the 5 position,

$R^4$ and $R^5$ are alkyl groups having 1 to 4 carbon atoms or together with the nitrogen atom form a piperidino, pyrrolidino or morpholino group,

$R^6$ is an alkyl group with 1 to 3 carbon atoms, and

n is 2 or 3,

and their acid additions salts.

2. Anilino-1,2,3-triazol-derivatives according to claim 1, having the general formula

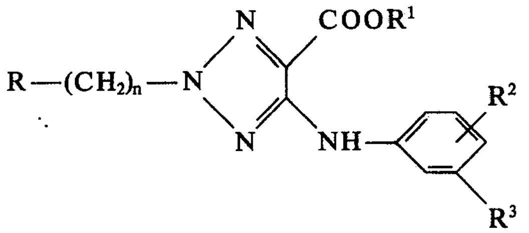

(Ia)

wherein

$R^1$ is a straight chain or a branched chain alkyl group with 1 to 4 carbon atoms,

$R^2$ is a hydrogen atom, a chlorine atom attached to the 3 or 4 position or a methyl group, a methoxy group, a nitro group, an amino group, a methoxy-carbonyl group, a fluorine atom or bromine atom attached to the 4-position,

$R^3$ is a hydrogen atom or a chlorine atom,

R is a dimethylamino group, a diethylamino group or a piperidyl-(1) or morpholino-(4) ring and

n is 1 or 2,

and their acid addition salts.

3. Anilino-1,2,3-triazol derivatives according to claim 2 of the general formula Ia, wherein

$R^1$ is a methyl group, an ethyl group or an n-butyl group, and

R, $R^2$, $R^3$ and n are as defined in claim 2,

and their acid addition salts.

4. Anilino-1,2,3-triazol derivatives according to claim 3, of the general formula Ia, wherein

$R^1$ is a methyl or n-butyl group,

$R^2$ is a methyl group or a chlorine or bromine atom attached to the 4-position,

$R^3$ is a hydrogen atom,

R is a diethylamino group or a dimethylamino group and

n is 2 or 3,

and their acid addition salts.

5. Process for the production of compounds according to claims 1 to 4, characterized in that a triazol derivative of the general formula

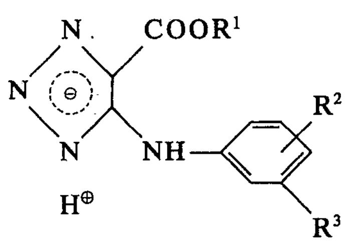

(III)

wherein $R^1$ to $R^3$ are as defined in the preceding claims, is reacted with a compound of the general formula

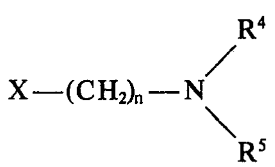

(IV)

wherein $R^4$, $R^5$ and n are as defined in the preceding claims and X is a halogen atom and the resulting compound is optionally converted into an acid addition salt.

6. A process according to claim 5, characterized in that the reaction is carried out in an aprotic polar solvent in the presence of a base.

7. Pharmaceutical preparations, characterized in that they contain one or more compounds according to claims 1 to 4 together with conventional carriers and excipients.

8. A process for the production of pharmaceutical preparations according to claim 7, characterized by formulating one or more compounds according to claim 1 to 4 with conventional carriers, excipients, disintegrants, or lubricants or substances for producing a delayed release, in the form of tablets, capsules, suppositories, powder or solution.

9. Compounds according to one of claims 1 to 4 for use in the treatment of blood flow disorders or sleep disorders.

10. The use of compounds according to one of claims 1 to 4 in the production of medicaments.

**Revendications**

1. Nouveaux dérivés d'anilino-1,2,3-triazole de formule générale

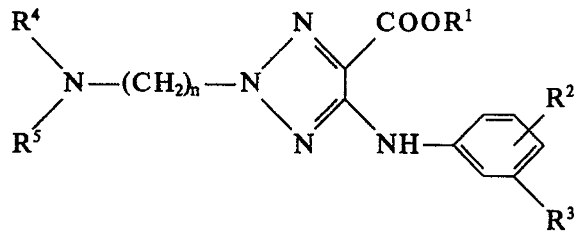

(I)

dans laquelle

$R^1$ représente un groupe alcoyle rectiligne ou ramifié avec 1 à 4 atomes de carbone,
$R^2$ représente un atome d'hydrogène, un atome d'halogène, un groupe amino, un groupe nitro, un groupe alcoxy acec 1 à 3 atomes de carbone, un groupe ester $COOR^6$ ou un groupe méthyle en position 3 ou 4,
$R^3$ représente un atome d'hydrogène ou un atome d'halogène en position 5,
$R^4$ et $R^5$ représentent des groupes alcoyle avec 1 à 4 atomes de carbone ou ensemble avec l'atome d'azote un cycle pipéridine, pyrrolidine ou morpholine,
$R^6$ représente un groupe alcoyle avec 1 à 3 atomes de carbone et
n représente le nombre 2 ou 3,

et leurs sels d'addition d'acides.

2. Dérivés d'anilino-1,2,3-triazole selon la revendication 1 de formule générale

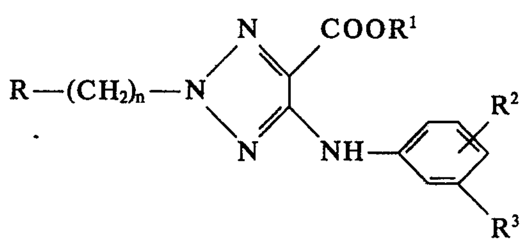

(Ia)

dans laquelle

$R^1$ représente un groupe alcoyle rectiligne ou ramifié avec 1 à 4 atomes de carbone,
$R^2$ représente un atome d'hydrogène, un atome de chlore en position 3 ou 4 ou un groupe méthyle, un groupe méthoxy, un groupe nitro, un groupe amino, un groupe méthoxycarbonyle, un atome de fluor ou de brome en position 4,
$R^3$ représente un atome d'hydrogène ou un atome de chlore,
R représente un groupe diméthylamino, un groupe diéthylamino ou un cycle pipéridyle-(1) ou morpholino-(4) et
n représente le nombre 2 ou 3,

ainsi que leurs sels d'addition d'acides.

3. Dérivés d'anilino-1,2,3-triazole selon la revendication 2 de formule générale Ia, dans laquelle

$R^1$ représente un groupe méthyle, un groupe éthyle ou un groupe n-butyle et
R, $R^2$, $R^3$ et n possèdent les significations indiquées dans la revendication 2 ainsi que leurs sels d'addition d'acides.

4. Dérivés d'anilino-1,2,3-triazole selon la revendication 3 de formule générale Ia, dans laquelle

$R^1$ représente un groupe méthyle ou n-butyle,
$R^2$ représente un groupe méthyle ou un atome de chlore ou de brome en position 4,
$R^3$ représente un atome d'hydrogène et
R représente un groupe diéthylamino ou diméthylamino et
n représente le nombre 2 ou 3,

ainsi que leurs sels d'addition d'acides.

5. Procédé pour la préparation de composés selon les revendications 1 à 4, caractérisé en ce qu'on fait réagir un dérivé de triazole de formule générale

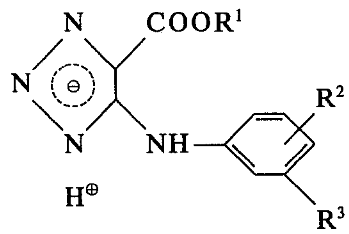

(III)

dans laquelle $R^1$ à $R^3$ possèdent les significations mentionnées plus haut, avec un composé de formule générale

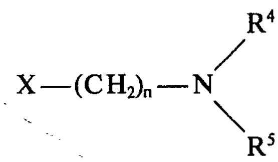

(IV)

dans laquelle $R^4$, $R^5$ et n sont définis comme indiqué plus haut et X représente un atome d'halogène et en ce qu'on transforme éventuellement le composé en un sel d'addition d'acide.

6. Procédé selon la revendication 5, caractérisé en ce qu'on effectue la réaction dans un solvant polaire, aprotique et en présence d'une base.

7. Préparations pharmaceutiques, caractérisées en ce qu'elles contiennent un ou plusieurs composés selon l'une des revendications 1 à 4 conjointement à des adjuvants et excipients usuels.

8. Procédé pour la fabrication de préparations pharmaceutiques selon la revendication 7, caractérisé en ce qu'on formule un ou plusieurs composés selon l'une des revendications 1 à 4 avec des adjuvants, excipients, désagrégeants ou diluants ou respectivement substances pour obtenir un effet retard usuels, en comprimés, capsules, suppositoires, poudres ou solutions.

9. Composés selon l'une des revendications 1 à 4 pour l'utilisation dans le traitement des perturbations de l'irrigation sanguine et du sommeil.

10. Utilisation de composés selon l'une des revendications 1 à 4 pour la préparation de médicaments.